# EUROPEAN PATENT SPECIFICATION

(11) **EP 2 769 664 B1**
(45) Date of publication and mention of the grant of the patent: **24.04.2019**
(21) Application number: 13156586.3
(22) Date of filing: 25.02.2013
(51) Int. Cl.: A61B 1/00, A61B 1/012

(54) **DISTAL PORTION OF RIGID ENDOSCOPE**
DISTALER ABSCHNITT EINES STARREN ENDOSKOPS
PARTIE DISTALE D'UN ENDOSCOPE RIGIDE

(43) Date of publication of application: 27.08.2014
(60) Divisional application: 19154626.6
(73) Proprietor: Integrated Medical Systems International, Inc., Birmingham, AL 35209 (US)
(72) Inventor: YE, Shusheng, Davie, FL 33328 (US); BODOR, Zoltan A., Plantation, FL 33325 (US); MCKENNA, Sean, Miami, FL 33136 (US); BODOR, Peter Pal, Pembroke Pines, FL 33029 (US)
(74) Representative: Range, Christopher William

(56) References cited:
- WO-A1-00/48505
- WO-A2-2006/105283
- US-A- 4 721 097
- US-A- 5 575 756
- US-A1- 2006 036 132

## Description

### Field of the Invention

The present invention is directed to a rigid endoscope, and more particularly, to a distal portion of a rigid endoscope.

### Background of the Invention

The distal portion of an autoclavable rigid endoscope typically consists of an inner tubular portion, an outer tubular portion and a composite of epoxy adhesive and optical fibers that fill in spaces in between the inner and outer tubular portions. During autoclave cycles the outer tube heats up and expands while the inner tube remains cool and does not expand. The difference in expansion results in shearing stresses that cause separation and failure of the epoxy adhesive joint.

### Summary of the Invention

The present invention is directed to a distal portion of a rigid endoscope including an inner tube and an outer tube fixed thereto using permanent means. By fixing the inner tube to the outer tube, thermally induced shear stress caused during autoclaving is reduced or eliminated.

According to the invention, there is provided an endoscope distal tip according to claim 1.

WO-A-2006/105283 discloses an arthroscope from which may be derived some features of claim 1 appended hereto.

A plurality of optical fibers extend longitudinally within a space formed between the outer endoscope tube and the inner endoscope tube. A distal end section of the plurality of optical fibers is divided into at least two spaced apart bundles of optical fibers. A tubular member having a tapered exterior surface is provided between the inner endoscope tube and the outer endoscope tube and distally to the connecting rib for aggregating the at least two spaced apart bundles of optical fiber into a single bundle of optical fibers. The tubular member may be provided between the inner tube and the plurality of optical fibers or between the plurality of optical fibers and the outer endoscope tube. Alternatively, a curved member can be used to aggregate the at least two bundles of optical fibers positioned between the outer endoscope tube and the plurality of optical fibers and distally to the connecting rib. In each instance, the tubular member and the curved member displace a portion of the plurality of optical fibers.

The endoscope distal tip may comprise a tubular assembly including a distal end, a proximal end, an outer tube, an inner tube, at least one rib rigidly coupling the outer tube to the inner tube, and a plurality of optical fibers provided between the outer tube and the inner tube, the plurality of optical fibers having a midsection divided into at least two optical fiber bundles and a distal end section provided as a single optical fiber bundle. The single optical fiber bundle is provided within a recess formed between the distal end of the tubular assembly and a distal end of the at least one rib. The proximal end of the tubular assembly is coupled to an endoscope optical subassembly including an optical tube having key hole shaped slots formed at both ends thereof, the optical tube being coupled between an objective housing and an ocular housing. The proximal end of the tubular assembly is coupled to an endoscope illumination subassembly including an illumination tube, a scope body and a flexible diaphragm coupled between the illumination tube and the scope body.

The invention provides a method of assembling an endoscope distal tip including providing an endoscope tip tubular assembly including an outer tube rigidly coupled to an inner tube by at least one rib extending there between, and providing a plurality of optical fibers within a space formed between the outer tube and the inner tube. The plurality of optical fibers are extended longitudinally and divided by the at least one rib into at least two optical fiber bundles. Thereafter, distal end sections of the at least two optical fiber bundles are aggregated into a single optical fiber bundle by inserting an aggregating member into a recess formed between a distal end of the at least one rib and a distal end of the endoscope tip tubular assembly. The aggregating member can be provided to encircle or partially encircle the inner tube or encircle or partially encircle the single optical fiber bundle.

### Brief Description of the Drawings

FIG. 1 is a plan view of a distal end of a distal portion of a 0° rigid endoscope in accordance with a preferred embodiment of the present invention.
FIG. 2 is a sectional view of the distal portion of the 0° rigid endoscope of FIG. 1 along line A-A.
FIG. 3 is a sectional view of the distal portion of the 0° rigid endoscope of FIG. 1 along line B-B.
FIG. 4 is an exploded view of an endoscope window assembly.
FIG. 5 is a perspective view of the endoscope window assembly of FIG. 4.
FIG. 6 is a sectional view of the endoscope window assembly of FIG. 4.
FIG. 7 is a perspective view of the distal end of the distal portion of the 0° rigid endoscope of FIG. 1 illustrating the endoscope window assembly of FIG. 4.
FIG. 8 is a sectional view of the distal portion of the 0° rigid endoscope of FIG. 7.
FIG. 9 is a side plan view of the distal portion of the 0° rigid endoscope of FIG. 7 illustrating coupling of an inner tube of the distal portion to an optical tube of an endoscope optical subassembly.
FIG. 10 is a plan view of the distal end of the distal portion of the 0° rigid endoscope of FIG. 9.
FIG. 11 is a sectional view of the distal portion of the 0° rigid endoscope and optical tube of FIG. 10 along line C-C.
FIG. 12 is a perspective view of the distal end of the distal portion of the 0° rigid endoscope of FIG. 9 illustrating coupling of an outer tube of the distal portion to an illumination tube of an endoscope illumination subassembly and the arrangement of a plurality of optical fibers therein.
FIG. 13 is a plan view of the distal end of the distal portion of the 0° rigid endoscope of FIG. 12.
FIG. 14 is a perspective view of an aggregating member in accordance with a preferred embodiment of the present invention.
FIG. 15 is a plan view of a distal end of the aggregating member of FIG. 14.
FIG. 16 is a sectional view of the aggregating member of FIG. 15 along line D-D.
FIG. 17 is sectional view of the distal portion of the 0° rigid endoscope of FIG. 12 illustrating the aggregating member of FIG. 14.
FIG. 18 is a perspective view of the distal end of the distal portion of the 0° rigid endoscope of FIG. 17.
FIG. 19 is a plan view of the distal end of the distal portion of the 0° rigid endoscope of FIG. 17.
FIG. 20 is a perspective view of a distal end of a distal portion of a 30° rigid endoscope in accordance with a preferred embodiment of the present invention.
FIG. 21 is a side plan view of the distal portion of the 30° rigid endoscope of FIG. 20.
FIG. 22 is a plan view of the distal end of the distal portion of the 30° rigid endoscope of FIG. 20.
FIG. 23 is a sectional view of the distal portion of the 30° rigid endoscope of FIG. 22 along line E-E.
FIG. 24 is a side plan view of the distal portion of the 30° rigid endoscope of FIG. 20 illustrating coupling of an outer tube of the distal portion with an illumination tube of an endoscope illumination subassembly.
FIG. 25 is a plan view of the distal end of the distal portion of the 30° rigid endoscope of FIG. 24 illustrating the arrangement of a plurality of optical fibers therein.
FIG. 26 is a partially exploded view of the distal end of the distal portion of the 30° rigid endoscope of FIG. 24 illustrating an aggregating member in accordance with a preferred embodiment of the present invention.
FIG. 27 is a perspective view of the distal end of the distal portion of the 30° rigid endoscope of FIG. 26 illustrating the aggregating member of FIG. 26.
FIG. 28 is a plan view of the distal end of the distal portion of the 30° rigid endoscope of FIG. 27.
FIG. 29 is a partially exploded view of an endoscope optical tube in accordance with a preferred embodiment of the present invention coupled between an objective housing and an ocular housing.
FIG. 30 is a perspective view of the optical tube of FIG. 29.
FIG. 31 is a sectional view of an endoscope including an optical tube of an endoscope optical subassembly flexibly coupled to a scope body in accordance with a preferred embodiment of the present invention.
FIG. 32 is a detail sectional view of the of a flexible diaphragm of the endoscope illumination subassembly of FIG. 31 coupling a scope body to an optical tube.
FIG. 33 is a perspective view of the flexible diaphragm of FIG. 31.
FIG. 34 is a sectional view of the flexible diaphragm of FIG. 33.
FIG. 35 is a perspective view of an alternatively designed flexible diaphragm.
FIG. 36 is a sectional view of the flexible diaphragm of FIG. 35.
FIG. 37 is a sectional view of the endoscope of FIG. 32 including an illumination subassembly coupled to an endoscope eyepiece of an endoscope optical subassembly in accordance with a preferred embodiment of the present invention.

### Detailed Description of Presently Preferred Embodiments

The present invention is directed an endoscope that is capable of withstanding harsh conditions associated with medical equipment cleaning and sterilization, particularly, the high temperatures and pressures associated with autoclaving. More particularly, the present invention is directed to a distal portion of a rigid endoscope including an inner tube and an outer tube rigidly fixed thereto using permanent means and various methods for assembling endoscopes including methods for installing optical fibers and assembling endoscope subassemblies.

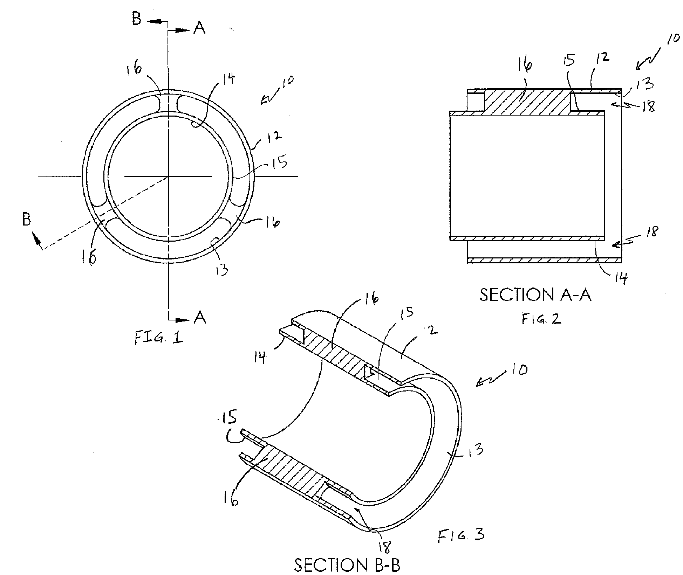

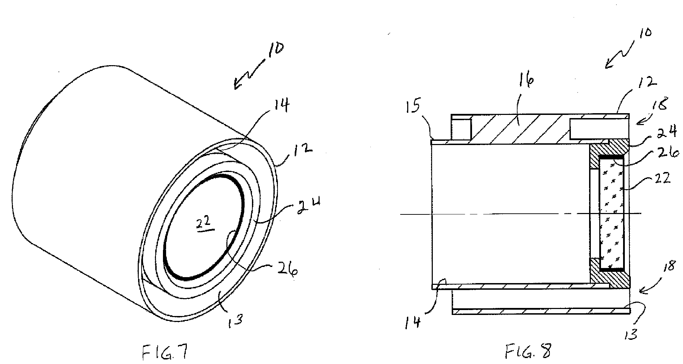

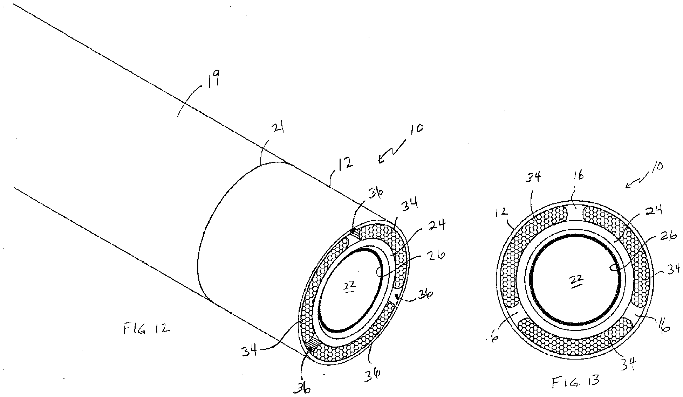

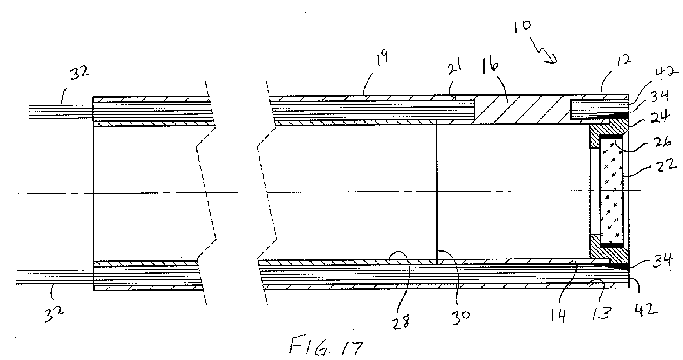

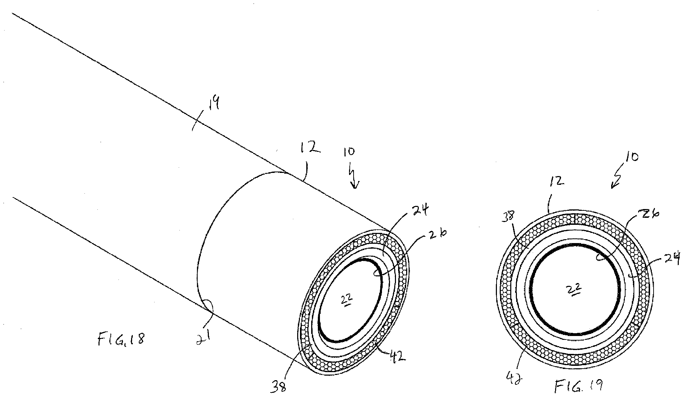

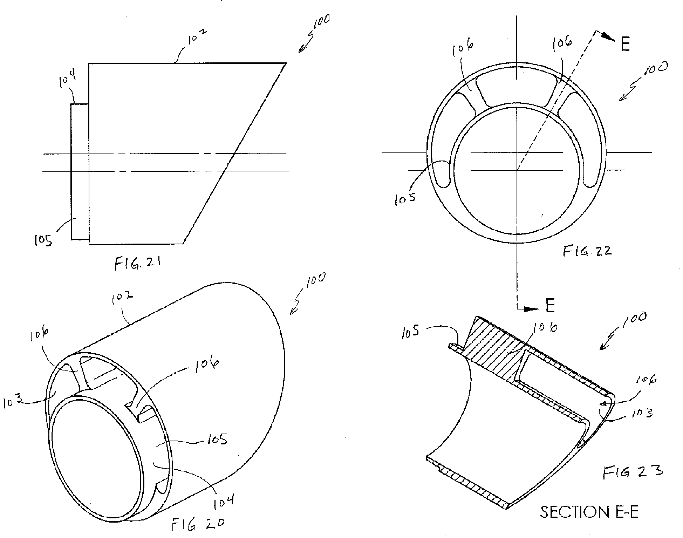

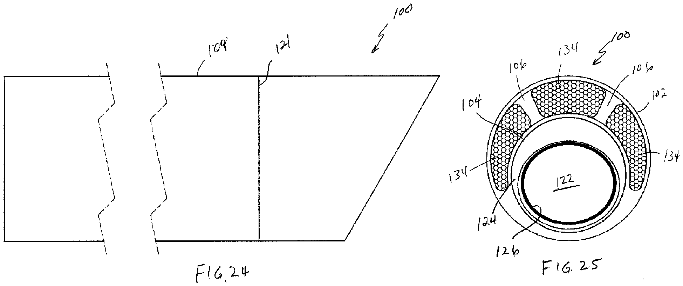

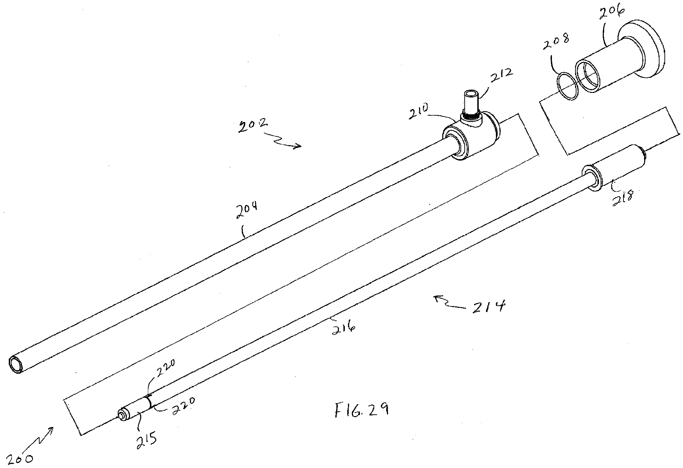

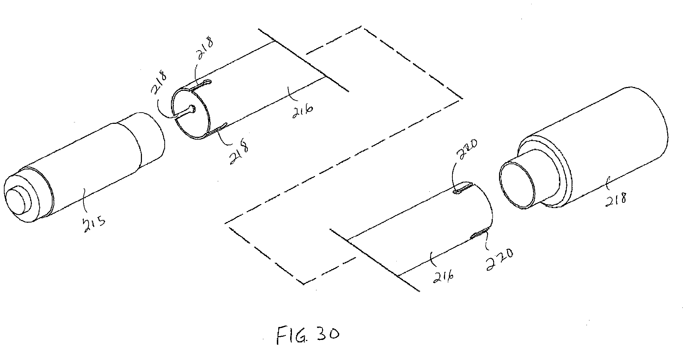

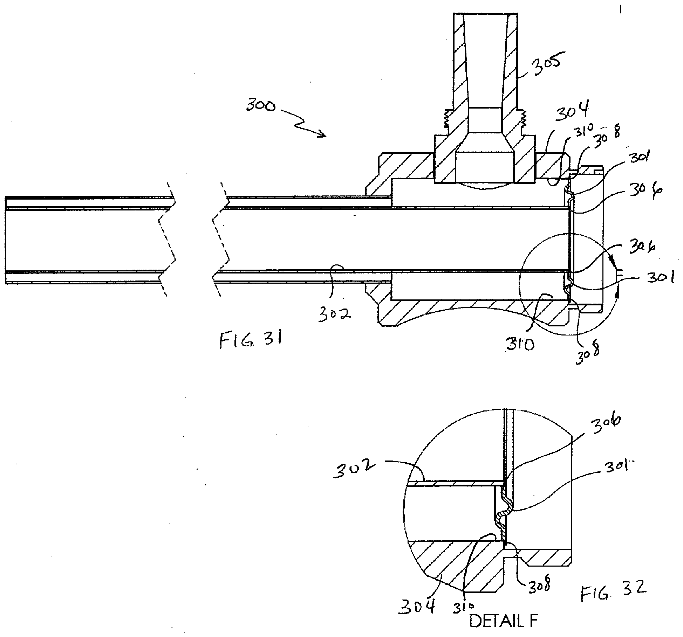

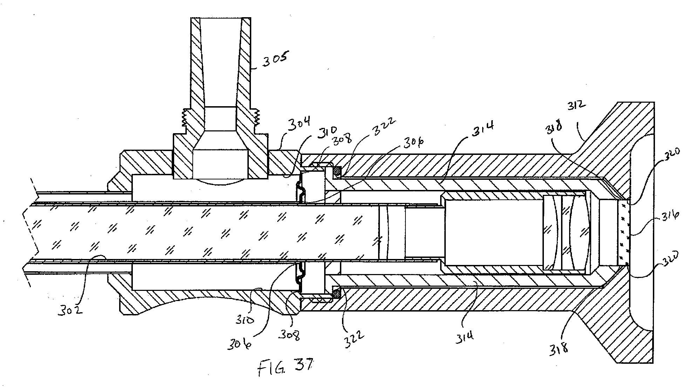

Referring to the drawings, FIGS. 1 through 19 illustrate a distal portion of a 0° rigid endoscope in accordance with a preferred embodiment of the present invention. FIGS. 20 through 28 illustrate a distal portion of a 30° rigid endoscope in accordance with a preferred embodiment of the present invention. FIGS. 29 and 30 illustrate an optical tube in accordance with a preferred embodiment of the present invention for use in an endoscope optical subassembly. FIGS. 31 through 36 illustrate a means of flexibly coupling an endoscope optical tube of an endoscope optical subassembly to a scope body in accordance with a preferred embodiment of the present invention. FIG. 37 illustrates an endoscope illumination subassembly coupled to an endoscope eyepiece of an endoscope optical subassembly in accordance with a preferred embodiment of the present invention.

Referring to the drawings, FIGS. 1 through 19, there is depicted a distal portion 10 of a 0° rigid endoscope including an outer tube 12, an inner tube 14, and three ribs 16 which extend to and between outer tube 12 and inner tube 14 for permanently and rigidly coupling tubes 12 and 14 together. Outer tube 12 is cylindrically-shaped having a inner surface 13. Inner tube 14 is cylindrically-shaped, having an outer surface 15. Inner tube 14 is concentrically arranged within outer tube 12 and recessed within a distal end thereof, while extending outwardly from a proximal end of outer tube 12. Ribs 16 are equidistantly spaced about inner tube 14 and extend from outer surface 15 of inner tube 14 to inner surface 13 of outer tube 12. Ribs 16 have a rectangular cross-section and are recessed from the distal end of outer tube 12 thereby forming a recess 18. It is contemplated that ribs 16 can be flush with the distal end of outer tube 12. Distal portion 10 can be manufactured using known machining techniques such as milling or wire electric discharge machining (EDM).

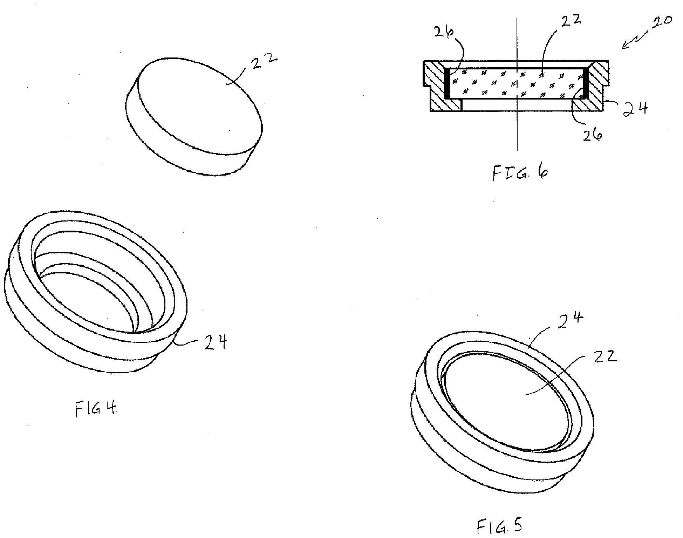

Referring to FIGS. 4 through 10, there is depicted a window assembly 20 for insertion into the distal end of distal portion 10. Window assembly 20 includes a window 22 constructed of sapphire or other suitable material that is brazed directly to a window seat 24 made of titanium or similar material along a joint 26. Window assembly 20 is inserted into the distal end of distal portion 10, and window seat 24 is directly coupled to inner tube 14 by soldering or brazing window seat 24 to the distal end of inner tube 14. Preferably, the solder or braze temperature is less than the temperature used to braze window 22 within window seat 24.

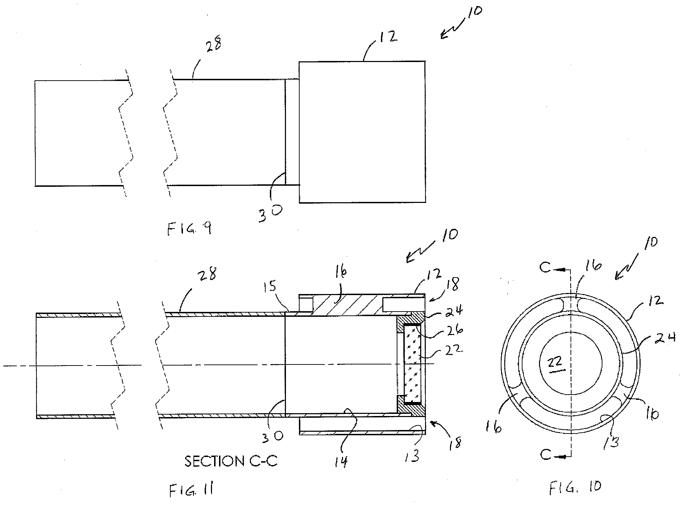

Referring to FIGS. 9 through 11, following insertion of window assembly 20 into distal portion 10, an endoscope optical tube 28 of an endoscope optical tube assembly is welded to the proximal end of inner tube 14 along a joint 30. A plurality of optical fibers 32 is then extended through the space formed between inner surface 13 of outer tube 12 and outer surface 15 of inner tube 14 from the proximal end of distal portion 10 to be flush with the distal end of outer tube 12. As plurality of optical fibers 32 are inserted into distal portion 10, the fibers are diverted around and to each side of each of ribs 16 thereby dividing the midsection and distal end of plurality of optical fibers 32 into three optical fiber bundles 34 separated by gaps 36. The width of each of gaps 36 is substantially equal to the width of each of ribs 16. Thereafter, an illumination tube 19 of an endoscope illumination subassembly is laser welded to a proximal end of outer tube 12 along joint 21 with plurality of optical fibers 32 extending there through.

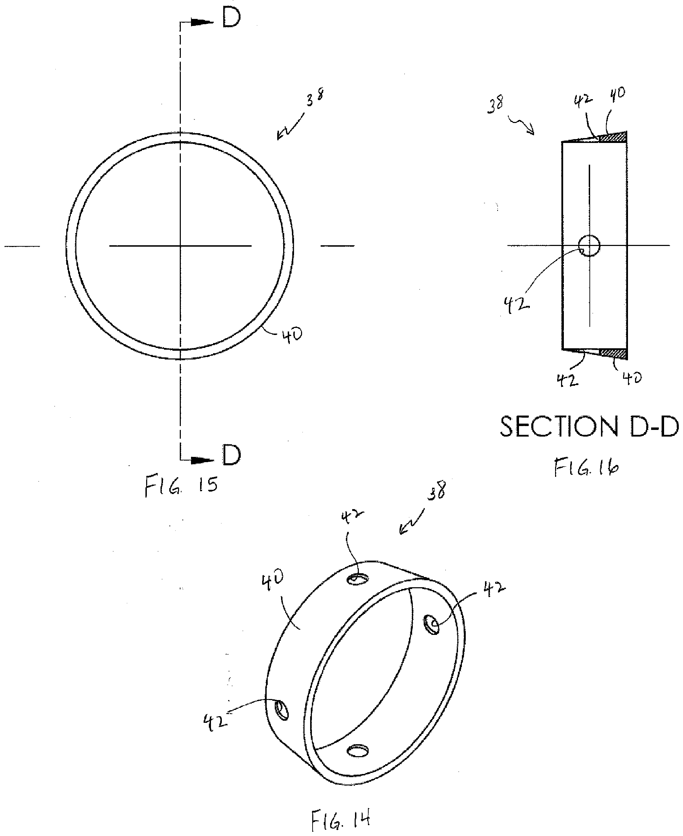

Referring FIGS. 14 through 16, there is depicted an aggregating member 38 for aggregating the three optical fiber bundles 34 at the distal end of plurality of optical fibers 32 into a single optical fiber bundle in order to provide even lighting at the tip of distal portion 10 when in use. Aggregating member 38 is cylindrically-shaped having a constant inner diameter that matches the outer diameter of inner tube 14, a tapered exterior surface 40 thereby providing the sidewall thereof to have a variable thickness, and a plurality of glue holes 42 through the sidewall. To aggregate the three optical fiber bundles 34 into a single optical fiber bundle, aggregating member 38 is axially aligned with inner tube 14 with thin end of the sidewall of aggregating member 38 facing distal portion 10. Aggregating member 38 is then pressed onto inner tube 14 and into recess 18 while epoxy is applied to the three optical fiber bundles 34. As aggregating 38 member is pressed onto inner tube 14, tapered exterior surface 40 rides against the interior surfaces of the three optical fiber bundles 34. As the thicker potion of the aggregating member 38 sidewall advances along the three optical fiber bundles 34, the optical fibers are displaced by aggregating member 38 and forced into gaps 36. In so doing, the three optical fiber bundles 34 are merged into a single optical fiber bundle 44.

Referring to the drawings, FIGS. 21 through 28, there is depicted a distal portion 100 of a 30° rigid endoscope including an outer tube 102, an inner tube 104, and two ribs 106 which extend to and between outer tube 102 and inner tube 104 for permanently and rigidly coupling tubes 102 and 104 together. Outer tube 102 is cylindrically-shaped having a inner surface 103. Inner tube 104 is cylindrically-shaped, having an outer surface 105. Inner tube 104 is provided within outer tube 102 is an offset arrangement so that inner tube 104 and outer tube 102 share a sidewall portion 107. The distal end of inner tube 104 is flush with the distal end of outer tube 102, while the proximal end of inner tube 104 extends outwardly from a proximal end of outer tube 102. Ribs 106 are spaced about inner tube 104 and extend from outer surface 105 of inner tube 104 to inner surface 103 of outer tube 102. Ribs 106 have a rectangular cross-section and are recessed from the distal end of outer tube 102 thereby forming a recess 108. Distal portion 100 can be manufactured using known machining techniques such as milling or wire EDM

As in distal portion 10, distal portion 100 includes a window assembly for insertion into the distal end of distal portion 100. The window assembly includes a window 122 that is brazed directly to a window seat 124 along a joint 126. The window assembly is inserted into the distal end of distal portion 100, and window seat 124 is directly coupled to inner tube 104 by soldering or brazing window seat 124 to the distal end of inner tube 104. Further, as in distal portion 10, following insertion of the window assembly into distal portion 100, an endoscope optical tube of an endoscope optical tube assembly is laser welded to the proximal end of inner tube 14 along a joint. A plurality of optical fibers is then extended through the space formed between inner surface 103 of outer tube 102 and outer surface 105 of inner tube 104 from the proximal end of distal portion 100 to be flush with the distal end of outer tube 102. As the plurality of optical fibers are inserted into distal portion 100, the fibers are diverted around and to each side of each of ribs 106 thereby dividing the midsection and distal end of the plurality of optical fibers into three optical fiber bundles 134 separated by gaps 136. The width of each of gaps 136 is substantially equal to the width of each of ribs 106.

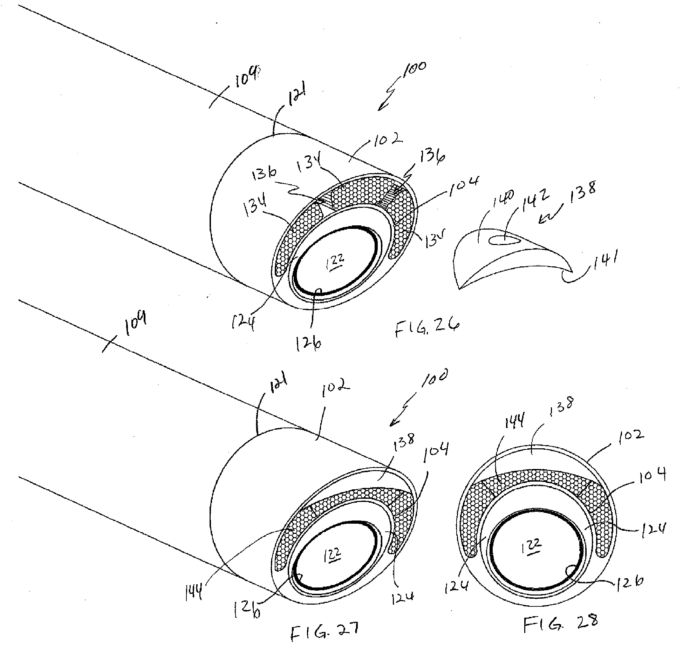

Referring FIGS. 26 through 28, there is depicted an aggregating member 138 for aggregating the three optical fiber bundles 134 at the distal end of the plurality of optical fibers into a single optical fiber bundle in order to provide even lighting at the tipoff distal portion 100 when in use. Aggregating member 138 is wedge-shaped having a curved exterior surface 140 matching the curvature of inner surface 103 of outer tube 102, a curved interior surface 141 having a less pronounced curve than exterior surface 140 thereby providing aggregate member 138 a crescent-shaped cross-section, a tapered sidewall thickness having a thicker distal side than proximal side, and a glue hole 142. To aggregate the three optical fiber bundles 134 into a single optical fiber bundle, aggregating member 138 is aligned with recess 106 with thin side of the sidewall of aggregating member 138 facing distal portion 100. Aggregating member 138 is then pressed into recess 106 with curved exterior surface 140 pressed against inner surface 103 of outer tube 102 while epoxy is applied to the three optical fiber bundles 134. As aggregating member 138 is pressed into recess 106, interior surface 141 rides against the exterior surfaces of the three optical fiber bundles 134. As the thicker potion of the aggregating member 138 sidewall advances along the three optical fiber bundles 134, the optical fibers are displaced by aggregating member 138 and forced into gaps 136. In so doing, the three optical fiber bundles 134 are merged into a single optical fiber bundle 144.

Referring to FIGS. 29 and 30, there is a depicted an endoscope illumination and optical assembly 200 in accordance with a preferred embodiment of the present invention. Assembly 200 includes an illumination subassembly 202 having an illumination tube 204, an eye piece 206, a sealing element 208, a scope body 210 and a light cone 212. Assembly 200 further includes an optical subassembly 214 including an optical tube 216, an objective housing 215, and an ocular housing 218, all of which are concentrically contained within illumination subassembly 202. Each end of optical tube 216 includes a plurality of keyhole shaped slots 220 which may be cut by a laser of other machining means. Objective housing 215 is lightly press-fitted into the distal end of optical tube 216. The elastic properties imparted to the distal end of optical tube 216 by the inclusion of key hole shaped slots 220 are used to maintain objective housing 215 in place temporarily to allow minor adjustments before housing 215 is glued in place. Slots 220 also provide larger surface areas for the glue to seep in between optical tube 216 and objective housing 215 thereby improving adherence of the joint there between. Similarly, the elastic properties imparted to the proximal end of optical tube 216 by the inclusion of key hole shaped slots 220 are used to maintain ocular housing 218 in place temporarily to allow minor adjustments before housing 218 is glued in place. Slots 220 also provide larger surface areas for the glue to seep in between optical tube 216 and ocular housing 218 thereby improving adherence of the joint there between. Optical system components such as rod lenses and spacer are loaded into optical tube 216.

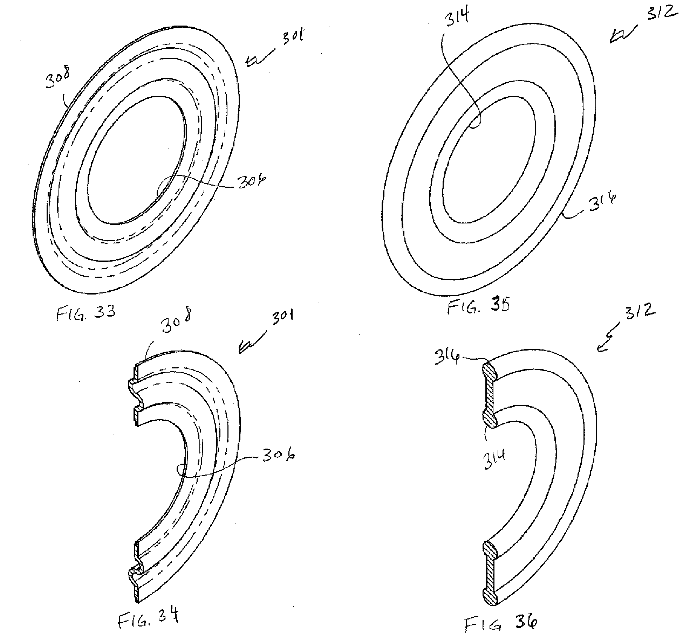

Referring to FIGS. 31 and 36, there is depicted an endoscope 300 including a flexible diaphragm 301 for coupling an endoscope optical tube 302 of an endoscope optical subassembly to a scope body 304 having a light cone 305. Diaphragm 301 permits deflection of optical tube 302 in the axial direction and thus relieves thermal expansion and stresses in optical tube 302 during sterilization. As depicted in FIGS. 34 and 36, diaphragm 300 can be provided as a wavy steel washer having inner circular edge 306 that is welded about and to the proximal end of optical tube 302 and its outer circular edge 308 welded to an interior surface 310 of scope body 304. Alternatively, diaphragm 301 may be a silicone rubber washer 312 with a dumbbell shaped cross-section as depicted in FIGS. 36 and 38. An inner circular edge 314 of diaphragm 312 can be slipped over the proximal end of optical tube 302 and sandwiched by thrust nuts. An outer edge 316 of diaphragm 312 can be similarly attached to scope body 304.

Referring to FIG. 37, there is depicted endoscope 300 illustrating an eyepiece housing 302 coupled to endoscope optical tube 302 and scope body 304. Eyepiece housing 312 contains a window housing 314 arranged concentrically therein. At the proximal end of eyepiece housing 314, a window 316 is soldered to window housing 314 at joint 318 and eyepiece housing 312 at joint 320. At the distal end of eyepiece housing 312, window housing 314 is welded to optical tube 302 and the proximal end of scope body 304 is coupled to and enclosed by eyepiece housing 312. A sealing element 322 is provided between scope body 304 and eyepiece housing 312.

As will be apparent to one skilled in the art, various modifications can be made within the scope of the appended claims. Such modifications being within the ability of one skilled in the art form a part of the present invention and are embraced by the claims below.

## Claims

1. An endoscope distal tip (10) comprising:
a tubular assembly including a distal end, a proximal end, an outer endoscope tube (12), an inner endoscope tube (14), and at least one connecting rib (16) extending to and between the outer endoscope tube and the inner endoscope tube,
wherein the at least one rib rigidly couples the inner endoscope tube to the outer endoscope tube,
the distal tip further comprising a plurality of optical fibres (32) extending longitudinally within a space formed between the outer endoscope tube (12) and the inner endoscope tube (14),
and by the distal tip further comprising an aggregating member (38, 138) positioned between the inner endoscope tube (14) and the outer endoscope tube (12) and distally to the connecting rib (16), the aggregating member (38, 138) displacing a portion of the plurality of optical fibres, wherein the aggregating member comprises a tubular member (38) having a tapered exterior surface (40) that is provided between the inner endoscope tube (14) and the outer endoscope tube (12) and distally to the connecting rib (16) wherein the tubular member is provided between the inner endoscope tube and a plurality of optical fibers (44) contained within the outer endoscope tube.

2. An endoscope distal tip (10) comprising:
a tubular assembly including a distal end, a proximal end, an outer endoscope tube (12), an inner endoscope tube (14), and at least one connecting rib (16) extending to and between the outer endoscope tube and the inner endoscope tube,
wherein the at least one rib rigidly couples the inner endoscope tube to the outer endoscope tube,
the distal tip further comprising a plurality of optical fibres (32) extending longitudinally within a space formed between the outer endoscope tube (12) and the inner endoscope tube (14),
and by the distal tip further comprising an aggregating member (38, 138) positioned between the inner endoscope tube (14) and the outer endoscope tube (12) and distally to the connecting rib (16), the aggregating member (38, 138) displacing a portion of the plurality of optical fibres,
wherein the aggregating member comprises a curved member (138) positioned between the outer endoscope tube and the plurality of optical fibers and distally to the connecting rib (16), the curved member displacing a portion of the plurality of optical fibers.

3. The endoscope distal tip according to claim 1 or claim 2 wherein the plurality of optical fibers includes a midsection divided into at least two spaced apart bundles (34) of optical fibers and a distal end section where the plurality of optical fibers are combined to form a single bundle (44) of optical fibers.

4. The endoscope distal tip according to claim 1, 2 or 3, wherein the outer endoscope tube (12) is coupled to an endoscope illumination subassembly and the inner endoscope tube (14) is coupled to an endoscope optical subassembly, the endoscope illumination subassembly including an illumination tube (305), a scope body (304) and a flexible diaphragm (301, 312) coupled between the illumination tube (305) and the scope body (304) and the endoscope optical subassembly (302) including an optical tube (302) coupled between the inner endoscope tube and an ocular housing.

5. An endoscope optical subassembly comprising an endoscope distal tip according to claim 1, 2, 3 or 4
an optical tube (302),
a scope body (304), and
a flexible diaphragm (301, 312) coupled to and between the optical tube and the scope body.

6. The endoscope optical subassembly according to claim 5 wherein optical tube (302) extends within an illumination tube that is coupled to the scope body (304).

7. The endoscope optical subassembly according to claim 6 wherein the optical tube (302) is movable axially along a longitudinal axis thereof relative to the illumination tube.

8. The endoscope optical subassembly according to claim 7 further comprising a light cone (305) and eyepiece assembly coupled to the scope body (304).

9. The endoscope optical subassembly according to any of claims 5 to 8 wherein the diaphragm (301, 312) is a wavy metal washer.

10. The endoscope optical subassembly according to any of claims 5 to 8 wherein the diaphragm (301, 312) is constructed of rubber.

11. The endoscope optical assembly according to any of claims 5 to 10 wherein the diaphragm (301, 312) has an inner edge (306) coupled to and encircling a proximal end of the optical tube (302) and an outer edge (308) coupled to the scope body (304).

12. The endoscope optical assembly according to any of claims 5 to 11 wherein the diaphragm (312) has a dumbbell-shaped cross-section or a substantially S-shaped cross-section portion.

## Patentansprüche

1. Distale Spitze (10) eines Endoskops, umfassend:
einen rohrförmigen Aufbau mit einem distalen Ende, einem proximalen Ende, einer äußeren Endoskopröhre (12), einer inneren Endoskopröhre (14), und wenigstens einer Verbindungsrippe (16), welche sich bis zu und zwischen der äußeren Endoskopröhre und inneren Endoskopröhre ausstreckt,
wobei die wenigstens eine Rippe die innere Endoskopröhre starr mit der äußeren Endoskopröhre verbindet,
die distale Spitze eine Vielzahl optischer Fasern (32), die sich longitudinal in einem Raum zwischen der äußeren Endoskopröhre (12) und der inneren Endoskopröhre (14) ausbreiten, aufweist,
und an der distalen Spitze ein Sammelelement (38, 138) aufweist, positioniert zwischen der inneren Endoskopröhre (14) und äußeren Endoskopröhre (12), und distal zu der Verbindungsrippe (16), wo das Sammelelement (38, 138) einen Anteil der Vielzahl optischer Fasern verdrängt, wobei das Sammelelement ein Röhrenelement (38) mit einer spitz zulaufenden äußeren Oberfläche (40) aufweist, das zwischen der inneren Endoskopröhre (14) und der äußeren Endoskopröhre (12) und distal zur Verbindungsrippe (16) vorgesehen ist, wobei das Röhrenelement zwischen der inneren Endoskopröhre und einer Vielzahl von optischen Fasern (44) vorgesehen ist, die von der äußeren Endoskopröhre umfasst werden.

2. Distale Spitze (10) eines Endoskops, umfassend:
einen rohrförmigen Aufbau mit einem distalen Ende, einem proximalen Ende, einer äußeren Endoskopröhre (12), einer inneren Endoskopröhre (14), und wenigstens einer Verbindungsrippe (16), welche sich bis zu und zwischen der äußeren Endoskopröhre und inneren Endoskopröhre ausstreckt,
wobei die wenigstens eine Rippe die innere Endoskopröhre starr mit der äußeren Endoskopröhre verbindet,
die distale Spitze eine Vielzahl optischer Fasern (32), die sich longitudinal in einem Raum zwischen der äußeren Endoskopröhre (12) und der inneren Endoskopröhre (14) ausbreiten, aufweist,
und an der distalen Spitze ein Sammelelement (38, 138) aufweist, positioniert zwischen der inneren Endoskopröhre (14) und äußeren Endoskopröhre (12), und distal zu der Verbindungsrippe (16), wo das Sammelelement (38, 138) einen Anteil der Vielzahl optischer Fasern verdrängt,
wobei das Sammelelement ein gebogenes Element (138) aufweist, welches zwischen der äußeren Endoskopieröhre und der Vielzahl optischer Fasern und distal zur Verbindungsrippe (16) positioniert ist und einen Anteil der Vielzahl optischer Fasern verdrängt.

3. Distale Spitze eines Endoskops nach einem der Ansprüche 1 und 2, **dadurch gekennzeichnet, dass** die Vielzahl optischer Fasern einen Mittelteil aufweist, der in mindestens zwei räumlich getrennte Bündel (34) optischer Fasern aufgeteilt ist, sowie ein distaler Endbereich, an dem die Vielzahl optischer Fasern zusammengefasst werden zu einem einzelnen Bündel (44) optischer Fasern.

4. Distale Spitze eines Endoskops nach einem der Ansprüche 1 bis 3, **dadurch gekennzeichnet, dass** die äußere Endoskopröhre (12) an eine Endoskop-Beleuchtungsbaugruppe gekoppelt ist und die innere Endoskopröhre (14) an eine Endoskop-Optikbaugruppe gekoppelt ist, wobei die Endoskop-Beleuchtungsbaugruppe eine Beleuchtungsröhre (305), ein Rahmenkörper (304) und eine flexible Membran (301,312), gekoppelt zwischen der Beleuchtungsröhre (305), dem Rahmenkörper (304) und der Endoskop-Beleuchtungsbaugruppe (302), einschließlich eines optischen Tubus (302), gekoppelt mit der inneren Endoskopröhre und einem Okulargehäuse.

5. Eine Endoskop-Optikbaugruppe, welche aufweist:
eine distale Spitze eines Endoskops nach einem der Ansprüche 1 bis 4;
eine optische Röhre (302);
ein Rahmenkörper (304), und;
eine flexible Membran (301,312) gekoppelt an und zwischen der optischen Röhre und dem Rahmenkörper.

6. Eine Endoskop-Optikbaugruppe nach Anspruch 5, **dadurch gekennzeichnet, dass** die optische Röhre (302) sich zu einer Beleuchtungsröhre, die an den Rahmenkörper (304) gekoppelt ist, erstreckt.

7. Eine Endoskop-Optikbaugruppe nach Anspruch 6, **dadurch gekennzeichnet, dass** die optische Röhre (302) entlang einer Longitudinal-Achse relativ zur Beleuchtungsröhre in Richtung der Longitudinal-Achse bewegbar ist.

8. Eine Endoskop-Optikbaugruppe nach Anspruch 7, **dadurch gekennzeichnet, dass** sie einen Lichtkegel (305) und eine Okularbaugruppe, die an den Rahmenkörper (304) gekoppelt ist, aufweist.

9. Eine Endoskop-Optikbaugruppe nach einem der Ansprüche 5 bis 8, **dadurch gekennzeichnet, dass** die Membran (301, 312) eine gewellte Metallbeilagscheibe ist.

10. Eine Endoskop-Optikbaugruppe nach einem der Ansprüche 5 bis 8, **dadurch gekennzeichnet, dass** die Membran aus Kautschuk aufgebaut ist.

11. Eine Endoskop-Optikbaugruppe nach einem der Ansprüche 5 bis 10, **dadurch gekennzeichnet, dass** die Membran (301, 312) einen inneren Rand (306), der mit dem proximalen Ende der optischen Röhre (302) gekoppelt ist und es umschließt, und einen äußeren Rand (308), der an den Rahmenkörper (304) gekoppelt ist, aufweist.

12. Eine Endoskop-Optikbaugruppe nach einem der Ansprüche 5 bis 11, **dadurch gekennzeichnet, dass** die Membran (312) einen hantelförmigen Querschnitt oder einen im Wesentlichen S-förmigen Teilquerschnitt aufweist.

## Revendications

1. Pointe distale d'endoscope (10) comprenant :
- un ensemble tubulaire comportant une extrémité distale, une extrémité proximale, un tube d'endoscope externe (12), un tube d'endoscope interne (14), et au moins une nervure de liaison (16) s'étendant jusqu'à et entre le tube d'endoscope externe et le tube d'endoscope interne,
dans laquelle l'au moins une nervure couple rigidement le tube d'endoscope interne au tube d'endoscope externe,
la pointe distale comprenant en outre une pluralité de fibres optiques (32) s'étendant longitudinalement à l'intérieur d'un espace formé entre le tube d'endoscope externe (12) et le tube d'endoscope interne (14),
et par la pointe distale comprenant en outre un élément d'agrégation (38, 138) positionné entre le tube d'endoscope interne (14) et le tube d'endoscope externe (12) et distalement jusqu'à la nervure de liaison (16), l'élément d'agrégation (38, 138) déplaçant une partie de la pluralité de fibres optiques, dans laquelle l'élément d'agrégation comprend un élément tubulaire (38) ayant une surface extérieure effilée (40) qui est prévu entre le tube d'endoscope interne (14) et le tube d'endoscope externe (12) et distalement jusqu'à la nervure de liaison (16) dans laquelle l'élément tubulaire est prévu entre le tube d'endoscope interne et une pluralité de fibres optiques (44) contenues à l'intérieur du tube d'endoscope externe.

2. Pointe distale d'endoscope (10) comprenant :
- un ensemble tubulaire comportant une extrémité distale, une extrémité proximale, un tube d'endoscope externe (12), un tube d'endoscope interne (14), et au moins une nervure de liaison (16) s'étendant jusqu'à et entre le tube d'endoscope externe et le tube d'endoscope interne,
dans laquelle l'au moins une nervure couple rigidement le tube d'endoscope interne au tube d'endoscope externe,
la pointe distale comprenant en outre une pluralité de fibres optiques (32) s'étendant longitudinalement à l'intérieur d'un espace formé entre le tube d'endoscope externe (12) et le tube d'endoscope interne (14),
et par la pointe distale comprenant en outre un élément d'agrégation (38, 138) positionné entre le tube d'endoscope interne (14) et le tube d'endoscope externe (12) et distalement jusqu'à la nervure de liaison (16), l'élément d'agrégation (38, 138) déplaçant une partie de la pluralité de fibres optiques,
dans laquelle l'élément d'agrégation comprend un élément incurvé (138) positionné entre le tube d'endoscope externe et la pluralité de fibres optiques et distalement jusqu'à la nervure de liaison (16), l'élément incurvé déplaçant une partie de la pluralité de fibres optiques.

3. Pointe distale d'endoscope selon la revendication 1 ou la revendication 2 dans laquelle la pluralité de fibres optiques comporte une section centrale divisée en au moins deux faisceaux (34) de fibres optiques espacés les uns des autres et une section d'extrémité distale où la pluralité de fibres optiques est combinée pour former un seul faisceau (44) de fibres optiques.

4. Pointe distale d'endoscope selon la revendication 1, 2 ou 3, dans laquelle le tube d'endoscope externe (12) est couplé à un sous-ensemble d'éclairage d'endoscope et le tube d'endoscope interne (14) est couplé à un sous-ensemble optique d'endoscope, le sous-ensemble d'éclairage d'endoscope comportant un tube d'éclairage (305), un corps de scope (304) et un diaphragme souple (301, 312) couplé entre le tube d'éclairage (305) et le corps de scope (304) et le sous-ensemble optique d'endoscope (302) comportant un tube optique (302) couplé entre le tube d'endoscope interne et un boîtier oculaire.

5. Sous-ensemble optique d'endoscope comprenant une pointe distale d'endoscope selon la revendication 1, 2, 3 ou 4
un tube optique (302),
un corps de scope (304), et
un diaphragme souple (301, 312) couplé à et entre le tube optique et le corps de scope.

6. Sous-ensemble optique d'endoscope selon la revendication 5 dans lequel le tube optique (302) s'étend à l'intérieur d'un tube d'éclairage qui est couplé au corps de scope (304).

7. Sous-ensemble optique d'endoscope selon la revendication 6 dans lequel le tube optique (302) est mobile axialement le long d'un axe longitudinal de celui-ci par rapport au tube d'éclairage.

8. Sous-ensemble optique d'endoscope selon la revendication 7 comprenant en outre un cône de lumière (305) et un ensemble d'oculaire couplé au corps de scope (304).

9. Sous-ensemble optique d'endoscope selon l'une quelconque des revendications 5 à 8 dans lequel le diaphragme (301, 312) est une rondelle métallique ondulée.

10. Sous-ensemble optique d'endoscope selon l'une quelconque des revendications 5 à 8 dans lequel le diaphragme (301, 312) est fabriqué à partir de caoutchouc.

11. Ensemble optique d'endoscope selon l'une quelconque des revendications 5 à 10 dans lequel le diaphragme (301, 312) a un bord interne (306) couplé à et encerclant une extrémité proximale du tube optique (302) et un bord externe (308) couplé au corps de scope (304).

12. Ensemble optique d'endoscope selon l'une quelconque des revendications 5 à 11 dans lequel le diaphragme (312) a une section transversale en forme d'haltère ou une partie de section transversale sensiblement en forme de S.
